# EUROPÄISCHE PATENTSCHRIFT

(11) **EP 3 429 982 B1**
(45) Veröffentlichungstag und Bekanntmachung des Hinweises auf die Patenterteilung: **10.06.2020**
(21) Anmeldenummer: 17709715.1
(22) Anmeldetag: 14.03.2017
(51) Int. Cl.: C07C 29/56, C07C 45/28, C07C 33/03, C07C 47/21

(54) **VERFAHREN ZUR HERSTELLUNG VON PRENOL UND PRENAL AUS ISOPRENOL**
METHODS FOR PREPARING PRENOL AND PRENAL FROM ISOPRENOL
PROCEDE DE PRODUCTION DE PRENOL ET DE PRENAL A PARTIR D'ISOPRENOL

(30) Priorität: 15.03.2016 EP 16160410
(43) Veröffentlichungstag der Anmeldung: 23.01.2019
(73) Patentinhaber: BASF SE, 67056 Ludwigshafen am Rhein (DE)
(72) Erfinder: DEHN, Martine, 67056 Ludwigshafen (DE); BRUNNER, Bernhard, 67056 Ludwigshafen (DE); EBEL, Klaus, 69517 Gorxheimertal (DE); HUBER, Sabine, 67056 Ludwigshafen (DE)
(86) Internationale Anmeldenummer: PCT/EP2017/055922
(87) Internationale Veröffentlichungsnummer: WO 2017/157897

(56) Entgegenhaltungen:
- WO-A1-2008/037693
- WO-A1-2009/106621
- WO-A1-2009/106622

## Beschreibung

### HINTERGRUND DER ERFINDUNG

Die vorliegende Erfindung betrifft ein Verfahren zur Herstellung von 3-Methyl-2-butenol (Prenol) und 3-Methyl-2-butenal (Prenal) aus 3-Methyl-3-butenol (Isoprenol). Das Verfahren kann kontinuierlich oder batchweise durchgeführt werden.

### STAND DER TECHNIK

3-Methyl-2-butenol (Prenol) und 3-Methyl-2-butenal (Prenal) stellen wichtige Wertstoffe dar, da sie als Synthesebausteine in vielen großtechnischen Herstellungsverfahren eingesetzt werden. Beispielsweise eignen sie sich insbesondere als Ausgangsstoffe zur Herstellung isoprenoider Verbindungen. So werden Prenol und Prenal unter anderem zur Herstellung von Riechstoffen, wie beispielsweise Citral, oder Vitaminen, wie beispielsweise Vitamin E und Vitamin A, verwendet.

Im Stand der Technik sind diverse Verfahren zur Herstellung von Prenol bzw. Prenal bekannt.

Die WO 2008/037693 beschreibt ein mehrstufiges Verfahren zur Herstellung von Citral, bei dem unter anderem Prenol durch Isomerisierung von 3-Methyl-3-butenol (Isoprenol) an einem heterogenen Edelmetallkatalysator in Gegenwart von Wasserstoff und Prenal über eine oxidative Dehydrierung von Isoprenol in der Gasphase und nachgeschalteter basischer Isomerisierung gewonnen wird. Konkret wird bei dem Verfahren zur Herstellung von Prenol als heterogener Edelmetallkatalysator ein Pd enthaltender Katalysator auf keramischem Träger verwendet, der zusätzlich Selen enthält, wobei Prenol in einer Selektivität von 91 % bei einem Umsatz von 55 %, bezogen auf das eingesetzte Isoprenol erhalten wird. Prenal wird über eine oxidative Dehydrierung in Gegenwart eines sauerstoffhaltigen Gases an einem geträgerten Silber-Katalysator bei einer Temperatur von 450 °C durchgeführt, wobei Prenal in einer Selektivität von 75,6 %, bei einem Umsatz von 62,8 %, bezogen auf das eingesetzte Isoprenol, erhalten wird. Das beschriebene Verfahren hat den Nachteil, dass sich bei der Isomerisierung von Isoprenol Isoamylalkohol bildet, der nur schwer aus dem Reaktionsgemisch abgetrennt werden kann. Zudem ist die zur Herstellung von Prenal verwendete oxidative Dehydrierung in der Gasphase aufwendig und kostenintensiv und führt aufgrund der hohen Reaktionstemperaturen nur zu unzureichenden Selektivitäten.

Die WO 2009/106622 beschreibt ein Verfahren, bei dem die Isomerisierung olefinisch ungesättigter Alkohole unter wesentlich milderen Bedingungen gelingt, indem sie an einem Kohlenstoff-geträgerten edelmetallhaltigen Katalysator in sauerstoffhaltiger Atmosphäre durchgeführt wird. Trotz der milden Bedingungen gelingt die Isomerisierung olefinisch ungesättigter Alkohole in guten Ausbeuten und Selektivitäten, d. h. die Bildung von Nebenprodukten, insbesondere der entsprechenden gesättigten Alkohole, kann gering gehalten werden. Konkret beschrieben wird die teilweise Isomerisierung von 3-Methyl-3-butenol (Isoprenol) zu 3-Methyl-2-butenol (Prenol) bei Temperaturen von 80 °C in Gegenwart eines Pd-Katalysators auf Kohle an Luft unter erhöhtem Druck, wobei neben einer Mischung aus Isoprenol und Prenol auch zu einem geringen Anteil 3-Methyl-2-butenal (Prenal) gebildet wird.

Verfahren zur Herstellung olefinisch ungesättigter Carbonylverbindungen durch oxidative Dehydrierung ungesättigter Alkohole, bei denen die oxidative Dehydrierung unter milderen Bedingungen in der Flüssigphase durchgeführt wird, sind beispielsweise aus ACS Catal. 2011, 1, 636-640; Catalysis Communications 2009, 10(11), 1542; Journal of Catalysis 2008, 258(2) und 315; Green Chemistry 2009, 11 (1), 109, bekannt. In diesen Verfahren werden insbesondere Palladium-Katalysatoren eingesetzt.

Im Stand der Technik ist ebenfalls der Einsatz verschiedener Ruthenium-Katalysatoren beschrieben, welche eine Oxidation von allylischen Alkoholen unter milderen Bedingungen ermöglichen. Beispielsweise beschreiben Larock und Veraprath in J. Org. Chem. 1984, 49, 3435-3436, die Herstellung ungesättigter Aldehyde, u. A. auch von Prenal, durch Oxidation der entsprechenden allylischen Alkohole bei 70 °C in 1,2-Dichlorethan in Gegenwart von 10 Gew.-% RuO₂-Hydrat und einer Sauerstoffatmosphäre sowie in Gegenwart des Antioxidationsmittels 2,6-Di-tert-butyl-p-cresol.

Im Stand der Technik ist ebenfalls beschrieben, dass sich Prenol in den bekannten oxidativen Dehydrierungsverfahren üblicherweise wesentlich leichter zu Prenal oxidieren lässt als Isoprenol. Beispielsweise beschreiben Parlett et al., ACS Catal. 2011, 1, 636-640, ein Verfahren zur oxidativen Dehydrierung allylischer Alkohole unter milden Bedingungen in Gegenwart verschiedener Pd-Katalysatoren auf mesoporösem Siliciumdioxid und Luftsauerstoff. Unter anderem gelang ihnen die Oxidation von Prenol in Toluol bei 90 °C in Gegenwart von 0,5 Gew.-% eines Pd-Katalysators auf mesoporösem SiO₂, wobei nach 24 h Prenal in einer Selektivität von 90 % bei einem Umsatz von 27 % erhalten wurde. Hingegen erweist sich Isoprenol unter diesen Reaktionsbedingungen als unreaktiv.

Die WO 2009/106621 beschreibt ein Verfahren zur Herstellung olefinisch ungesättigter Carbonylverbindungen durch oxidative Dehydrierung ungesättigter Alkohole in sauerstoffhaltiger Atmosphäre bei Temperaturen im Bereich von 50 bis 240 °C und in Gegenwart eines Gold-Katalysators auf einem basischen Träger, der neben Gold gegebenenfalls weitere Edelmetalle enthält. Konkret wird die Herstellung von 3-Methyl-2-butenal (Prenal) aus 3-Methyl-3-butenol (Isoprenol) beschrieben, wobei aufgrund des Einsatzes basischer Trägermaterialien bevorzugt 3-Methyl-2-butenal (Prenal) und nicht 3-Methyl-3-butenal (Isoprenal) gebildet wird und damit keine nachgeschaltete Isomerisierung durchgeführt werden muss. Zusätzlich lehrt die WO 2009/106621, dass bei der Verwendung von Isoprenol/Prenol-Mischungen als Ausgangsstoff für die oxidative Dehydrierung die Umsatzrate gesteigert werden kann. Bei diesem Verfahren werden allerdings Verdünnungsmittel, wie beispielsweise Xylol, eingesetzt, was die Attraktivität für technische Verfahren reduziert.

Es besteht der Bedarf an einem verbesserten Verfahren zur Herstellung von Prenal und Prenol aus Isoprenol, womit die oben beschriebenen Nachteile, die sich aus der geringen Reaktivität von Isoprenol gegenüber der oxidativen Dehydrierung ergeben, überwunden werden können.

Der vorliegenden Erfindung liegt somit die Aufgabe zugrunde, ein Verfahren bereitzustellen, welches die Herstellung von 3-Methyl-2-butenol (Prenol) und 3-Methyl-2-butenal (Prenal) aus 3-Methyl-3-butenol (Isoprenol) unter milden Reaktionsbedingungen, insbesondere niedrigen Reaktionstemperaturen, ermöglicht, bei dem trotz der geringen Reaktivität von Isoprenol gegenüber oxidativer Dehydrierung hohe Umsatzraten erzielt werden können. Dabei soll das 3-Methyl-2-butenol (Prenol) und 3-Methyl-2-butenal (Prenal) in Form von Zusammensetzungen erhalten werden, die Prenol und Prenal in möglichst ähnlichen molaren Mengen enthalten. Zudem soll das Verfahren die Herstellung von Prenol und Prenal in hoher Gesamtselektivität ermöglichen, wobei die Bildung schwer abtrennbarer Nebenprodukte möglichst gering gehalten werden soll. Das Verfahren sollte sich zudem für eine kontinuierliche Verfahrensführung eignen.

Diese Aufgabe wird überaschenderweise gelöst durch ein Verfahren zur Herstellung einer Zusammensetzung, die 3-Methyl-2-butenol und 3-Methyl-2-butenal enthält, bei dem man
i) 3-Methyl-3-butenol einer katalytischen Isomerisierung an einem Kohlenstoff-geträgerten Pd-Katalysator in Gegenwart eines Gasgemisches enthaltend 1 bis 15 Vol.-% Sauerstoff unterzieht, unter Erhalt eines ersten Produktgemisches, das wenigstens 40 Gew.-% 3-Methyl-2-butenol, wenigstens 5 Gew.-% 3-Methyl-3-butenol und 0 bis 15 Gew.-% 3-Methyl-2-butenal, bezogen auf das Gesamtgewicht des ersten Produktgemisches, enthält,
ii) das in Schritt i) erhaltene erste Produktgemisch einer oxidativen Dehydrierung an einem Pd-Katalysator, der SiO₂ und/oder Al₂O₃ als Trägermaterial enthält, oder an einem Kohlenstoff-geträgerten Pd/Au-Katalysator in Gegenwart eines Gasgemisches enthaltend 5 bis 25 Vol.-% Sauerstoff unterzieht, unter Erhalt eines gegenüber dem ersten Produktgemisch an 3-Methyl-2-butenal angereicherten und 3-Methyl-2-butenol abgereicherten zweiten Produktgemisches, wobei das molare Verhältnis von 3-Methyl-2-butenol zu 3-Methyl-2-butenal in dem zweiten Produktgemisch im Bereich von 75 : 25 bis 35 : 65 liegt,
wobei die oxidative Dehydrierung in Schritt ii) zusätzlich in Gegenwart einer Base durchgeführt wird.

Es wurde gefunden, dass ein Reaktionsaustrag aus einer katalytischen Isomerisierung von Isoprenol ohne Zwischenreinigung, gegebenenfalls nach Abtrennung des Katalysators, in vorteilhafter Weise direkt in der oxidativen Dehydrierung eingesetzt werden kann. Die Reaktivität, d. h. der Umsatz, der in der oxidativen Dehydrierung eingesetzten Isomerisierungsausträge ist dabei vergleichbar mit dem Einsatz von reinem Prenol, wobei in der Summe sogar höhere Selektivitäten für Prenol und Prenal erreicht werden.

Des Weiteren wurde gefunden, dass die Reaktivität und Selektivität in der oxidativen Dehydrierung durch Basenzusätze zusätzlich gesteigert werden kann.

Ein weiterer Gegenstand der Erfindung betrifft die kontinuierliche Durchführung des Verfahrens wie zuvor und im Folgenden definiert.

### BESCHREIBUNG DER ERFINDUNG

Das erfindungsgemäße Verfahren weist wenigstens einen der folgenden Vorteile auf:
- Das Verfahren ermöglicht die Herstellung von Prenol und Prenal in hohen Gesamtselektivitäten.
- Mit dem erfindungsgemäßen Verfahren können auch unter milden Reaktionsbedingungen hohen Umsatzraten erzielt werden, wodurch die Bildung schwer abtrennbarer Nebenprodukte weitgehend oder sogar vollständig vermieden werden kann.
- Mit dem erfindungsgemäßen Verfahren können Zusammensetzungen erhalten werden, die Prenol und Prenal in ähnlichen molaren Mengen enthalten.
- Das Verfahren ist trotz der zweistufigen Reaktionsführung einfach durchführbar, da insbesondere aufwendige Zwischenreinigungen nicht erforderlich sind.
- Bei dem Verfahren kann weitgehend oder vollständig auf den Einsatz externer Verdünnungsmittel verzichtet werden, was in Verbindung mit den hohen Umsatzraten zu wesentlich verbesserten Raum-Zeit Ausbeuten führt.
- Das Verfahren kann in vorteilhafter Weise kontinuierlich durchgeführt werden.
- Das erfindungsgemäße Verfahren eignet sich aufgrund der oben genannten Merkmale zur Herstellung von Prenol und Prenal in großtechnischem Maßstab und zeichnet sich zudem durch eine vorteilhafte Wirtschaftlichkeit aus.

### Schritt i):

In Schritt i) des erfindungsgemäßen Verfahrens wird 3-Methyl-3-butenol (Isoprenol) zu 3-Methyl-2-butenol (Prenol) isomerisiert. Die Isomerisierung erfolgt erfindungsgemäß an einem Kohlenstoff-geträgerten Pd-Katalysator in Gegenwart eines Gasgemisches, enthaltend 1 bis 15 Vol.-% Sauerstoff.

Die katalytische Isomerisierung kann sowohl in flüssiger Phase als auch in der Gasphase durchgeführt werden, wobei die katalytische Isomerisierung bevorzugt in flüssiger Phase erfolgt.

Die katalytische Isomerisierung erfolgt üblicherweise in Gegenwart eines Gasgemisches, enthaltend 1 bis 15 Vol.-% Sauerstoff, d. h. in Gegenwart einer sauerstoffhaltigen Atmosphäre. Das Gasgemisch, enthaltend 1 bis 15 Vol.-% Sauerstoff, kann beispielsweise durch einmaliges Aufpressen vor oder zu Beginn der Reaktion, durch mehrfaches Aufpressen während der Reaktion oder durch kontinuierliche Einleitung über den gesamten Reaktionsverlauf der Reaktionszone zugeführt werden. Bevorzugt wird in Schritt ii) des erfindungsgemäßen Verfahrens das sauerstoffhaltige Gasgemisch durch kontinuierliches Einleiten über den gesamten Reaktionsverlauf der Reaktionszone zugeführt.

Üblicherweise enthält das in Schritt i) des erfindungsgemäßen Verfahren eingesetzte Gasgemisch einen Sauerstoffgehalt im Bereich von 1 bis 15 Vol.-%, vorzugsweise von 2 bis 12 Vol.-% und insbesondere von 3 bis 10 Vol.-%.

In einer besonders bevorzugten Ausführungsform des Schrittes i) des erfindungsgemäßen Verfahrens wird die sauerstoffhaltige Atmosphäre durch Zuführen eines an Sauerstoff angereicherten Gasgemisches und Abführen eines an Sauerstoff abgereicherten Gasgemisches ausgetauscht. Die Zuführung und Abführung des an Sauerstoff angereicherten bzw. an Sauerstoff abgereicherten Gasgemisches kann dabei nacheinander oder simultan erfolgen. Bevorzugt wird der Austausch der sauerstoffhaltigen Atmosphäre mehrfach durchgeführt, wobei die Gesamtmenge an ausgetauschtem sauerstoffhaltigem Gasgemisch wenigstens dem Volumen der in der Reaktionszone befindlichen Gasmenge entspricht. Besonders bevorzugt entspricht die Gesamtmenge an ausgetauschtem sauerstoffhaltigem Gasgemisch dem mehrfachen Volumen, beispielsweise dem 10-fachen Volumen, der in der Reaktionszone befindlichen Gasmenge. Der Austausch kann dabei stufenweise oder kontinuierlich erfolgen. Bevorzugt erfolgt der Austausch der sauerstoffhaltigen Atmosphäre kontinuierlich.

Falls die sauerstoffhaltige Atmosphäre kontinuierlich ausgetauscht wird, liegt die Austauschrate des sauerstoffhaltigen Gasgemisches bei der katalytischen Isomerisierung üblicherweise im Bereich von 1 bis 200 L/h, insbesondere im Bereich von 3 bis 120 L/h, insbesondere im Bereich von 5 bis 80 L/h, bezogen auf 100 g des eingesetzten Isoprenols.

Nach erfolgter katalytischer Isomerisierung in Schritt i) wird ein erstes Produktgemisch erhalten, welches neben 3-Methyl-2-butenol (Prenol) sowohl 3-Methyl-3-butenol (Isoprenol) als auch gegebenenfalls geringe Mengen an 3-Methyl-2-butenal (Prenal) enthält.

In der Regel enthält das in Schritt i) des erfindungsgemäßen Verfahren erhaltene erste Produktgemisch wenigstens 40 Gew.-% 3-Methyl-2-butenol, wenigstens 5 Gew.-% 3-Methyl-3-butenol und 0 bis 15 Gew.-% 3-Methyl-2-butenal.

Bevorzugt enthält das in Schritt i) erhaltene erste Produktgemisch 40 bis 80 Gew.-%, besonders bevorzugt 45 bis 70 Gew.-% 3-Methyl-2-butenol.

Bevorzugt enthält das in Schritt i) erhaltene erste Produktgemisch 0 bis 15 Gew.-%, besonders bevorzugt 5 bis 10 Gew.-% 3-Methyl-2-butenal.

Bevorzugt enthält das in Schritt i) erhaltene erste Produktgemisch 5 bis 59 Gew.-%, besonders bevorzugt 20 bis 50 Gew.-% 3-Methyl-3-butenol.

In einer bevorzugten Ausführungsform des erfindungsgemäßen Verfahrens besteht das in Schritt i) erhaltene erste Produktgemisch aus
i.1 40 bis 80 Gew.-% 3-Methyl-2-butenol,
i.2 0 bis 15 Gew.-% 3-Methyl-2-butenal,
i.3 5 bis 59 Gew.-% 3-Methyl-3-butenol und
i.4 0 bis 10 Gew.-% von i.1, i.2 und i.3 verschiedenen Verbindungen.

In einer besonders bevorzugten Ausführungsform des erfindungsgemäßen Verfahrens besteht das in Schritt i) erhaltene erste Produktgemisch aus
i.1 45 bis 70 Gew.-% 3-Methyl-2-butenol,
i.2 5 bis 10 Gew.-% 3-Methyl-2-butenal,
i.3 20 bis 50 Gew.-% 3-Methyl-3-butenol und
i.4 0 bis 5 Gew.-% von i.1, i.2 und i.3 verschiedenen Verbindungen.

Der Gesamtanteil an 3-Methyl-2-butenol, 3-Methyl-2-butenal und 3-Methyl-3-butenol in dem ersten Produktgemisch beträgt dabei üblicherweise wenigstens 80 Gew.-%, bevorzugt wenigstens 85 Gew.-%, besonders bevorzugt wenigstens 90 Gew.-% oder mehr, beispielsweise wenigstens 92 Gew.-%, bezogen auf das Gesamtgewicht des ersten Produktgemisches.

Die Isomerisierung in Schritt i) findet erfindungsgemäß in Gegenwart eines Kohlenstoff-geträgerten Pd-Katalysator statt, d. h. einem Pd-Katalysator, der ein Trägermaterial auf Basis von Kohlenstoff (C), wie beispielsweise Aktivkohle, enthält. Nur beispielhaft seien für solche Materialien verschiedene Arten von Kohle oder verschiedene Arten von Graphit genannt, wie sie dem Fachmann bekannt und in der Literatur beschrieben sind.

Grundsätzlich eignen sich als Trägermaterialien auf Basis von Kohlenstoff alle dem Fachmann für derartige Verwendungen bekannten Kohlenstoffmaterialien. Die Trägermaterialien können vorzugsweise in Form von Formkörpern, Granulaten, Strängen, Pellets, Splitt, Tabletten oder Prills eingesetzt werden. Die Oberfläche der Trägermaterialien nach BET (25 °C) liegt üblicherweise im Bereich von 1 bis 10000, vorzugsweise von 10 bis 5000 m²/g, ist aber für das erfindungsgemäße Verfahren in den meisten Fällen nicht kritisch.

Der Pd-Gehalt des in der katalytischen Isomerisierung eingesetzten Kohlenstoff-geträgerten Pd-Katalysators liegt üblicherweise im Bereich von 0,1 bis 20 Gew.-%, bevorzugt im Bereich von 0,5 bis 15 Gew.-% und insbesondere im Bereich von 1 bis 10 Gew.-%, bezogen auf das Gesamtgewicht des Kohlenstoff-geträgerten Pd-Katalysators.

Üblicherweise liegt die Menge des in der katalytischen Isomerisierung eingesetzten Kohlenstoff-geträgerten Pd-Katalysators im Bereich von 0,1 bis 20 Gew.-%, bevorzugt im Bereich von 0,2 bis 10 Gew.-%, besonders bevorzugt im Bereich von 0,5 bis 5 Gew.-%, bezogen auf die Menge des in der Reaktionsmischung enthaltenen Isoprenols.

Die katalytischen Isomerisierung wird üblicherweise bei einem Druck im Bereich von 1 bis 100 bar, vorzugsweise im Bereich von 1 bis 50 bar, insbesondere im Bereich von 2 bis 20 bar durchgeführt.

Üblicherweise liegt die Reaktionstemperatur bei der katalytischen Isomerisierung im Bereich von 20 bis 150 °C, vorzugsweise im Bereich von 40 bis 120 °C und insbesondere im Bereich von 50 bis 110 °C.

Die katalytische Isomerisierung kann in Abwesenheit oder Anwesenheit eines externen Lösungsmittels erfolgen.

Falls die katalytische Isomerisierung in Anwesenheit eines externen Lösungsmittels erfolgt, handelt es sich dabei üblicherweise um ein inertes organisches Lösungsmittel. Im Rahmen der vorliegenden Erfindung ist unter einem inerten Lösungsmittel ein Lösungsmittel zu verstehen, welches unter den gewählten Reaktionsbedingungen keine Reaktion mit den an der Isomerisierung beteiligten Verbindungen und Stoffen eingeht.

Geeignete inerte Lösungsmittel sind beispielsweise aliphatische Kohlenwasserstoffe, wie Pentan, Hexan, Heptan, Ligroin, Petrolether oder Cyclohexan, halogenierten Kohlenwasserstoffe, wie Dichlormethan, Trichlormethan, Tetrachlormethan, aromatische Kohlenwasserstoffen, wie Benzol, Toluol, Xylole, halogenierten aromatischen Kohlenwasserstoffen, wie Chlorbenzol, Dichlorbenzolen, Ether, wie Diethylether, Methyl-tert.-Butylether, Dibutylether, Diphenylether, Tetrahydrofuran oder 1,4-Dioxan, sowie Mischungen davon. Besonders geeignet sind beispielsweise Toluol, o-Xylol, p-Xylol, Tetrahydrofuran, 1,4-Dioxan oder Diphenylether.

Bevorzugt wird die katalytische Isomerisierung ohne Zugabe eines externen Lösungsmittels durchgeführt.

Die katalytische Isomerisierung kann sowohl in Batch- als auch in kontinuierlicher Fahrweise durchgeführt werden. Bevorzugt wird die katalytische Isomerisierung in kontinuierlicher Fahrweise durchgeführt.

Üblicherweise wird die katalytische Isomerisierung von 3-Methyl-3-butenol zu 3-Methyl-2-butenol bis zu einem Umsatz von 30 bis 80 %, bevorzugt bis zu einem Umsatz von 35 bis 75 %, insbesondere von 40 bis 70 %, bezogen auf das eingesetzte 3-Methyl-3-butenol, geführt.

Bei dem zur katalytischen Isomerisierung eingesetzten 3-Methyl-3-butenol handelt es sich üblicherweise um kommerziell erhältliches 3-Methyl-3-butenol oder um 3-Methyl-3-butenol, welches bei der Wertstoffgewinnung, beispielsweise bei der Gewinnung von Citral, als Abfallstrom anfällt, sowie um Mischungen davon. Kommerziell erhältliches 3-Methyl-3-butenol kann im großtechnischen Maßstab, beispielsweise aus Formaldehyd und Isobuten, synthetisiert werden und stellt damit einen billigen und leicht zugänglichen chemischen Baustein dar.

Das in Schritt i) des erfindungsgemäßen Verfahrens erhaltene erste Produktgemisch wird nachfolgend auch als "Austrag der katalytischen Isomerisierung" bezeichnet. Das in Schritt i) des erfindungsgemäßen Verfahrens erhaltene erste Produktgemisch wird üblicherweise, gegebenenfalls nach Abtrennung des Katalysator gefolgt von einer weiteren Aufreinigung, beispielsweise einer destillativen Aufreinigung, oder gegebenenfalls nach Abtrennung des Katalysators ohne eine weitere Aufreinigung, in die oxidative Dehydrierung des Schrittes ii) eingesetzt, bzw. bei kontinuierlicher Fahrweise dem Schritt ii) zugeführt.

Bevorzugt wird der Austrag der katalytischen Isomerisierung, gegebenenfalls nach Abtrennung des Katalysators, ohne weitere Aufreinigung in die oxidative Dehydrierung des Schrittes ii) eingesetzt, bzw. bei kontinuierlicher Fahrweise dem Schritt ii) zugeführt.

### Schritt ii):

Die oxidative Dehydrierung gemäß Schritt ii) des erfindungsgemäßen Verfahrens wird in der Regel in Gegenwart eines Pd-Katalysators, der SiO₂, Al₂O₃ oder Mischungen aus SiO₂ und Al₂O₃ als Trägermaterial enthält, oder in Gegenwart eines Kohlenstoff-geträgerten Pd/Au-Katalysators durchgeführt.

Bevorzugt handelt es sich bei dem in der oxidativen Dehydrierung eingesetzten Pd-Katalysator um einen SiO₂- oder SiO₂/Al₂O₃-geträgerten Pd-Katalysator, insbesondere um einen SiO₂/Al₂O₃-geträgerten Pd-Katalysator.

Bevorzugt handelt es sich bei dem in der oxidativen Dehydrierung eingesetzten Kohlenstoff-geträgerten Pd/Au-Katalysator, d. h. einem Pd/Au-Katalysator, der ein Trägermaterial auf Basis von Kohlenstoff (C), wie beispielsweise Aktivkohle, enthält. Hinsichtlich der hierfür als Trägermaterial geeigneten Aktivkohlen wird auf die zuvor im Zusammenhang mit dem Kohlenstoff-geträgerten Pd-Katalysator gemachten Ausführungen Bezug genommen.

Der Metallgehalt des in Schritt ii) des erfindungsgemäßen Verfahrens eingesetzten Pd-Katalysators oder Pd/Au-Katalysators, unterliegt keiner besonderen Beschränkung. Bevorzugt liegt der Metallgehalt des in Schritt ii) des erfindungsgemäßen Verfahrens eingesetzt Pd-Katalysators oder Pd/Au-Katalysators im Bereich von 0,1 bis 25 Gew.-%, bevorzugt im Bereich von 0,5 bis 15 Gew.-% und insbesondere im Bereich von 2 bis 10 Gew.-%.

Der Pd-Gehalt des in Schritt ii) des erfindungsgemäßen Verfahrens eingesetzten Pd/Au-Katalysators liegt üblicherweise im Bereich von 0,5 bis 15 Gew.-%, bevorzugt im Bereich von 1 bis 10 Gew.-% und insbesondere im Bereich von 2 bis 7 Gew.-%, bezogen auf das Gesamtgewicht des Kohlenstoff-geträgerten Pd/Au-Katalysators.

Der Au-Gehalt des in Schritt ii) des erfindungsgemäßen Verfahrens eingesetzten Pd/Au-Katalysators liegt üblicherweise im Bereich von 0,1 bis 10 Gew.-%, bevorzugt im Bereich von 0,5 bis 7 Gew.-% und insbesondere im Bereich von 1 bis 5 Gew.-%, bezogen auf das Gesamtgewicht des Kohlenstoff-geträgerten Pd/Au-Katalysators.

Das Gewichtsverhältnis von Pd zu Au des in Schritt ii) eingesetzten Pd/Au-Katalysators liegt üblicherweise im Bereich von 1 : 50 bis 3 : 1, bevorzugt im Bereich von 1 : 20 bis 2 : 1, insbesondere im Bereich von 1 : 10 bis 1,2 : 1.

Die Herstellung der erfindungsgemäß eingesetzten SiO₂-, Al₂O₃- oder SiO₂/Al₂O₃-geträgerten Pd-Katalysatoren oder der erfindungsgemäß eingesetzten Kohlenstoff-geträgerten Pd/Au-Katalysatoren kann nach dem Fachmann bekannten und in der Literatur beschriebenen Verfahren erfolgen.

Üblicherweise liegt die Menge des in der oxidativen Dehydrierung eingesetzten Pd-Katalysators oder Pd/Au-Katalysators im Bereich von 0,1 bis 20 Gew.-%, bevorzugt im Bereich von 0,2 bis 10 Gew.-%, besonders bevorzugt im Bereich von 0,4 bis 5 Gew.-%, bezogen auf das Gesamtgewicht des Reaktionsgemisches der oxidativen Dehydrierung.

Die oxidative Dehydrierung gemäß Schritt ii) des erfindungsgemäßen Verfahrens wird bevorzugt in Gegenwart eines Pd-Katalysators, der SiO₂, Al₂O₃ oder Mischungen aus SiO₂ und Al₂O₃ als Trägermaterial enthält, durchgeführt.

Dementsprechend betrifft eine bevorzugte Ausführungsform der Erfindung ein Verfahren, wie oben definiert, bei dem in Schritt ii) ein Pd-Katalysator, der SiO₂ und/oder Al₂O₃ als Trägermaterial enthält, als Katalysator eingesetzt wird.

Hinsichtlich des bevorzugten und besonders bevorzugten Pd-Gehalts und/oder Trägermaterials sowie der bevorzugten und besonders bevorzugten Einsatzmengen des in dieser Ausführungsform in Schritt ii) eingesetzten Pd-Katalysators wird auf die oberen Ausführungen Bezug genommen.

Die oxidative Dehydrierung erfolgt üblicherweise in flüssiger Phase, d. h. der Druck und die Temperatur der Umsetzung werden so gewählt, dass die Einsatzstoffe in flüssiger Form vorliegen.

Die Temperatur bei der oxidativen Dehydrierung liegt vorzugsweise in einem Bereich von 10 bis 150 °C, bevorzugt im Bereich von 20 bis 130 °C, insbesondere im Bereich von 40 bis 120 °C.

Die oxidative Dehydrierung kann bei Umgebungsdruck oder vermindertem oder erhöhtem Druck erfolgen. Bevorzugt wird die oxidative Dehydrierung bei erhöhtem Druck durchgeführt. Besonders bevorzugt wird die oxidative Dehydrierung bei einem Druck im Bereich von 1 bis 200 bar, besonders bevorzugt von 1,5 bis 100 bar, insbesondere von 2 bis 50 bar, durchgeführt.

Üblicherweise erfolgt die oxidative Dehydrierung in Gegenwart eines Gasgemisches, enthaltend 5 bis 25 Vol.-% Sauerstoff, d. h. in Gegenwart einer sauerstoffhaltigen Atmosphäre.

Das in Schritt ii) des erfindungsgemäßen Verfahrens eingesetzte Gasgemisch, enthaltend 5 bis 25 Vol.-% Sauerstoff, kann beispielsweise durch einmaliges Aufpressen vor oder zu Beginn der Reaktion, durch mehrfaches Aufpressen während der Reaktion oder durch kontinuierliche Einleitung über den gesamten Reaktionsverlauf der Reaktionszone zugeführt werden. Bevorzugt wird in Schritt ii) des erfindungsgemäßen Verfahrens das sauerstoffhaltige Gasgemisch durch kontinuierliches Einleiten über den gesamten Reaktionsverlauf der Reaktionszone zugeführt.

Üblicherweise enthält das in Schritt ii) des erfindungsgemäßen Verfahrens eingesetzte Gasgemisch einen Sauerstoffgehalt im Bereich von 5 bis 25 Vol.-%, vorzugsweise von 7 bis 20 Vol.-% und insbesondere von 8 bis 15 Vol.-%.

In einer besonders bevorzugten Ausführungsform des Schrittes ii) des erfindungsgemäßen Verfahrens wird die sauerstoffhaltige Atmosphäre durch Zuführen eines an Sauerstoff angereicherten Gasgemisches und Abführen eines an Sauerstoff abgereicherten Gasgemisches ausgetauscht. Die Zuführung und Abführung des an Sauerstoff angereicherten bzw. an Sauerstoff abgereicherten Gasgemisches kann dabei nacheinander oder simultan erfolgen. Bevorzugt wird der Austausch der sauerstoffhaltigen Atmosphäre mehrfach durchgeführt, wobei die Gesamtmenge an ausgetauschtem sauerstoffhaltigem Gasgemisch wenigstens dem Volumen der in der Reaktionszone befindlichen Gasmenge entspricht. Besonders bevorzugt entspricht die Gesamtmenge an ausgetauschtem sauerstoffhaltigem Gasgemisch dem mehrfachen Volumen, beispielsweise dem 10-fachen Volumen, der in der Reaktionszone befindlichen Gasmenge. Der Austausch kann dabei stufenweise oder kontinuierlich erfolgen. Bevorzugt erfolgt der Austausch der sauerstoffhaltigen Atmosphäre kontinuierlich.

Falls die sauerstoffhaltige Atmosphäre kontinuierlich ausgetauscht wird, liegt die Austauschrate des sauerstoffhaltigen Gases oder Gasgemisches bei der oxidativen Dehydrierung üblicherweise im Bereich von 1 bis 200 L/h, bevorzugt im Bereich von 3 bis 120 L/h, insbesondere im Bereich von 5 bis 80 L/h, bezogen auf 100 g des in Schritt ii) eingesetzten ersten Produktgemisches.

Die oxidative Dehydrierung kann in Anwesenheit oder in Abwesenheit eines zugesetzten organischen Lösungsmittels durchgeführt werden.

Falls die oxidative Dehydrierung in Gegenwart eines zugesetzten organischen Lösungsmittels durchgeführt wird, handelt es sich dabei vorzugsweise um ein unter den Reaktionsbedingungen inertes organisches Lösungsmittel, wie bereits oben für die katalytische Isomerisierung definiert.

Bevorzugt wird bei der oxidativen Dehydrierung kein externes organisches Lösungsmittel zugesetzt.

Nach Beendigung der oxidativen Dehydrierung in Schritt ii) des erfindungsgemäßen Verfahrens wird in der Regel ein gegenüber dem ersten Produktgemisch aus Schritt i) an 3-Methyl-2-butenal angereichertes und an 3-Methyl-2-butenol abgereichertes zweites Produktgemisch erhalten.

Eine Anhebung wenigstens eines der Reaktionsparameter, insbesondere der Reaktionstemperatur, dem Druck, der Reaktionszeit bzw. Verweilzeit oder der Menge an eingesetztem Katalysator, führt in der Regel zu einer Umsatzsteigerung bzw. zu einem höheren Gehalt an 3-Methyl-2-butenal in der Reaktionsmischung der oxidativen Dehydrierung (zweites Produktgemisch). Allerdings ist aufgrund von Zersetzungs- und Folgereaktionen mit Selektivitätseinbußen zu rechnen. Insbesondere bei längeren Reaktionszeiten bzw. Verweilzeiten kann es zu einer Zunahme von Nebenreaktionen kommen, z. B. zur Überoxidation (vermehrte Bildung von Säuren, Epoxiden, Umlagerungsprodukten und dergleichen). Insbesondere kann dies zu einer Zunahme der Bildung von Isoamylalkohol führen, der nur schwer vom Reaktionsgemisch abgetrennt werden kann.

Aus diesem Grund werden bei der oxidativen Dehydrierung die Reaktionsparameter, insbesondere die Katalysatormenge, der Druck, die Reaktionstemperatur und die Reaktionszeit bzw. Verweilzeit, in der Regel so gewählt, dass der Anteil von 3-Methyl-2-butenal in dem zweiten Produktgemisch üblicherweise im Bereich von 2 bis 55 Gew.-%, bevorzugt im Bereich von 10 bis 45 Gew.-% und insbesondere im Bereich von 15 bis 35 Gew.-%, bezogen auf das Gesamtgewicht des zweiten Produktgemisches, liegt.

Die oxidative Dehydrierung in Schritt ii) des erfindungsgemäßen Verfahrens wird üblicherweise so geführt, dass das molare Verhältnis von 3-Methyl-2-butenol zu 3-Methyl-2-butenal in dem zweiten Produktgemisch im Bereich von 75 : 25 bis 35 : 65, bevorzugt im Bereich von 70 : 30 bis 40 : 60 und insbesondere im Bereich von 65 : 35 bis 45 : 55, liegt.

Das so erhaltene zweite Produktgemisch kann nach anschließender Abtrennung des eingesetzten Pd-Katalysators sowie nach einer destillativen Abtrennung wenigstens eines Teils des im zweiten Produktgemisch enthaltenen 3-Methyl-3-butenols direkt als Einsatzstoff zur Herstellung isoprenoider Wertstoffe, z. B. zur Herstellung von Riechstoffen, wie beispielsweise Citral, oder Vitaminen, wie beispielsweise Vitamin E und Vitamin A, verwendet werden.

Üblicherweise wird die oxidative Dehydrierung in Schritt ii) zusätzlich in Gegenwart einer Base durchgeführt. Geeignete Basen, die bei der oxidativen Dehydrierung eingesetzt werden können, sind Mineralbasen, insbesondere die Hydroxide der Alkali- und Erdalkalimetalle, wie beispielsweise LiOH, NaOH, KOH, RbOH, CsOH, Mg(OH)₂ oder Ca(OH)₂. Besonders bevorzugt ist die in der oxidative Dehydrierung eingesetzte Base ausgewählt unter NaOH und KOH.

Der Anteil der Base in der Reaktionsmischung liegt bevorzugt im Bereich von 0,01 bis 2 Gew.-%, besonders bevorzugt im Bereich von 0,05 bis 1,5 Gew.-%, insbesondere im Bereich von 0,1 bis 1 Gew.-%, bezogen auf die Menge des in Schritt ii) eingesetzten ersten Produktgemisches.

In einer speziellen Ausführungsform des erfindungsgemäßen Verfahrens wird die oxidative Dehydrierung des in Schritt i) erhaltenen ersten Produktgemisches, wie oben definiert, an einem SiO₂/Al₂O₃-geträgerten Pd-Katalysator oder an einem Kohlenstoff-geträgerten Pd/Au-Katalysator in Gegenwart eines Gasgemisches, enthaltend 8 bis 15 Vol.-% Sauerstoff, und in Gegenwart von 0,1 bis 1 Gew.-% KOH oder NaOH, bezogen auf die Menge des eingesetzten ersten Produktgemisches, sowie bei einer Temperatur im Bereich von 40 bis 120 °C und einem Druck im Bereich von 2 bis 50 bar, derart durchgeführt, dass ein zweites Produktgemisch erhalten wird, welches 3-Methyl-2-butenol und 3-Methyl-2-butenal in einem molaren Verhältnis im Bereich von 70 : 30 bis 40 : 60 enthält.

In einer besonders bevorzugten Ausführungsform der Schritte i) und ii) des erfindungsgemäßen Verfahrens liegt der Sauerstoffgehalt des in Schritt i) eingesetzten Gasgemisches 2 bis 10 Vol.-%, bevorzugt 3 bis 8 Vol.-%, insbesondere 4 bis 6 Vol.-%, unter dem Sauerstoffgehalt des in Schritt ii) eingesetzten Gasgemisches.

In einer weiteren besonders bevorzugten Ausführungsform des erfindungsgemäßen Verfahrens wird in den Schritten i) und ii) ein an Sauerstoff angereichertes Gasgemisch den jeweiligen Reaktionszonen kontinuierlich zugeführt und ein an Sauerstoff abgereichertes Gasgemisch kontinuierlich abgeführt. Die kontinuierliche Zuführung des an Sauerstoff angereicherten Gasgemisches zu den jeweiligen Reaktionszonen kann auf verschiedene Weisen erfolgen. Bevorzugt erfolgt die kontinuierliche Zuführung des an Sauerstoff angereicherten Gasgemisches unterhalb der Flüssigkeitsoberfläche der jeweiligen Reaktionsgemische.

In einer speziellen Ausführungsform des erfindungsgemäßen Verfahrens wird in den Schritten i) und ii) ein an Sauerstoff angereichertes Gasgemisch den jeweiligen Reaktionszonen kontinuierlich zugeführt und ein an Sauerstoff abgereichertes Gasgemisch kontinuierlich abgeführt, wobei die Zuführung des an Sauerstoff angereicherten Gasgemisches unterhalb der Flüssigkeitsoberfläche der jeweiligen Reaktionsgemische erfolgt.

### Destillative Auftrennung

In einer weiteren Ausführungsform des erfindungsgemäßen Verfahrens wird das in Schritt ii) erhaltene zweite Produktgemisch einer destillativen Auftrennung in eine an 3-Methyl-2-butenol und 3-Methyl-2-butenal angereicherte Produktfraktion und eine an 3-Methyl-3-butenol angereicherte Eduktfraktion unterzogen.

Zur destillativen Auftrennung des in Schritt ii) erhaltenen zweiten Produktgemisches eignen sich in der Regel alle Vorrichtungen zur destillativen Auftrennung von Reaktionsgemischen, die flüssige Komponenten enthalten. Geeignete Vorrichtungen umfassen Destillationskolonnen, wie Bodenkolonnen, die mit Glockenböden, Siebplatten, Siebböden, Packungen oder Füllkörpern ausgerüstet sein können, oder Drehbandkolonnen Verdampfer, wie Dünnschichtverdampfer, Fallfilmverdampfer, Zwangsumlaufverdampfer, Sambay-Verdampfer, etc. und Kombinationen davon. Besonders bevorzugt wird zur destillativen Auftrennung der in Schritt ii) erhaltenen Zusammensetzung Destillationskolonnen und/oder Drehbandkolonnen verwendet, insbesondere Drehbandkolonnen.

Bei der destillativen Auftrennung wird aus dem in Schritt ii) erhaltenen zweiten Produktgemisch zunächst ein Brüden abgezogen, der anschließend zumindest teilweise kondensiert wird. Zur Kondensation bzw. partiellen Kondensation des Brüden können alle geeigneten Kondensatoren verwendet werden. Diese können mit beliebigen Kühlmedien gekühlt werden. Kondensatoren mit Luftkühlung und/oder Wasserkühlung sind bevorzugt, wobei die Luftkühlung besonders bevorzugt ist.

Die nach der destillativen Auftrennung erhaltene an 3-Methyl-2-butenol und 3-Methyl-2-butenal angereicherte Produktfraktion kann anschließend, je nach gewünschtem Reinheitsgrad, weiteren Reinigungsschritten, wie Destillation oder den üblichen dem Fachmann geläufigen flüssigchromatographischen Verfahren, unterzogen werden. In der Regel kann die nach der destillativen Auftrennung erhaltene an 3-Methyl-2-butenol und 3-Methyl-2-butenal angereicherte Produktfraktion direkt zur Herstellung isoprenoider Wertstoffe, z. B. zur Herstellung von Riechstoffen, wie beispielsweise Citral, oder Vitaminen, wie beispielsweise Vitamin E und Vitamin A, verwendet werden.

Die nach der destillativen Auftrennung erhaltene an 3-Methyl-3-butenol angereicherte Eduktfraktion kann als Einsatzstoff in die katalytische Isomerisierung in Schritt i) eingesetzt werden. Zu dieser an 3-Methyl-3-butenol angereicherten Eduktfraktion wird hierzu gegebenenfalls frisches, d. h. reines kommerziell erhältliches 3-Methyl-3-butenol beigefügt. Falls die katalytische Isomerisierung kontinuierlich durchgeführt wird, wird zu der in Schritt i) eingesetzten an 3-Methyl-3-butenol angereicherten Eduktfraktion in der Regel frisches 3-Methyl-3-butenol zugegeben, wobei die zugegebene Menge an frischem 3-Methyl-3-butenol üblicherweise der Menge an frischem 3-Methyl-3-butenol entspricht, die erwartungsgemäß in der katalytischen Isomerisierung und der nachfolgenden oxidativen Dehydrierung des Schrittes ii) umgesetzt wird.

### Kontinuierliche Durchführung

Das erfindungsgemäße Verfahren kann batchweise oder in kontinuierlicher Fahrweise durchgeführt werden. Bevorzugt wird wenigstens einer der Schritte i) oder ii) des erfindungsgemäßen Verfahrens kontinuierlich durchgeführt. In einer besonders bevorzugten Ausführungsform des erfindungsgemäßen Verfahrens werden beide Schritte i) und ii) kontinuierlich durchgeführt.

Die Katalysatorbelastung bei der oxidativen Dehydrierung liegt üblicherweise im Bereich von 0,1 bis 50 kg 3-Methyl-2-butenal pro kg Katalysator und Stunde, vorzugsweise von 0,2 bis 30 kg 3-Methyl-2-butenal pro kg Katalysator und Stunde und insbesondere im Bereich von 0,5 bis 15 kg 3-Methyl-2-butenal pro kg Katalysator und Stunde.

Bei kontinuierlicher Fahrweise kann das erfindungsgemäße Verfahren in einem oder mehreren Reaktoren durchgeführt werden. Bevorzugt wird das Verfahren in wenigstens zwei Reaktoren durchgeführt, die miteinander in Form einer Kaskade verbunden sind.

Falls beide Verfahrensschritte kontinuierlich durchgeführt werden, werden die katalytische Isomerisierung und die oxidative Dehydrierung parallel, aber räumlich getrennt voneinander in jeweils wenigstens einem Reaktor, durchgeführt.

Beispielsweise erfolgt in einem ersten Reaktor die katalytische Isomerisierung wie oben definiert. Das so erhaltene erste Produktgemisch wird, gegebenenfalls unter Abtrennung des in fester Form vorliegenden Katalysators, als kontinuierlicher Produktstrom direkt der oxidativen Dehydrierung zugeführt, die parallel zur katalytischen Isomerisierung in einem zweiten Reaktor erfolgt. Gleichzeitig wird dem ersten Reaktor kontinuierlich 3-Methyl-3-butenol, wie oben definiert, zugeführt, um das Volumen der Reaktionslösung in dem ersten Reaktor konstant zu halten. Das nach der oxidativen Dehydrierung im zweiten Reaktor erhaltene gegenüber dem ersten Produktgemisch an 3-Methyl-2-butenal angereicherte und an 3-Methyl-2-butenol abgereicherte zweite Produktgemisch wird als Produktstrom dem zweiten Reaktor entnommen und kann als Einsatzstoff nach erfolgter Katalysatorabtrennung weiteren Verfahren, wie oben beschrieben, zugeführt werden.

Die Erfindung wird anhand der im Folgenden beschriebenen Beispiele näher erläutert. Dabei sollen die Beispiele nicht als einschränkend für die Erfindung verstanden werden.

In den nachfolgenden Beispielen werden folgende Abkürzungen verwendet:
SAbgasfahrweise steht für eine Reaktionsführung unter Austausch der sauerstoffhaltigen Atmosphäre, d. h. kontinuierliches Einleiten eines an Sauerstoff angereicherten (frischen) Gasgemisches unter gleichzeitiger kontinuierlicher Abführung eines an Sauerstoff abgereicherten (verbrauchten) Gasgemisches;
Abgas steht für die Menge an ausgetauschtem sauerstoffhaltigem Gasgemisch;
MBE steht für Isoprenol (3-Methyl-3-butenol);
SAP steht für einen SiO₂/Al₂O₃-geträgerten Pd-Katalysator.

### BEISPIELE

In den Beispielen wurden folgende Katalysatoren eingesetzt:

| | |
|---|---|
| 5 % Pd-C WET reduced (BASF-Italia) | Feuchter und vorreduzierter Pd-Katalysator der Firma BASF Italia mit Aktivkohle als Trägermaterial und einem Pd-Gehalt von 5 %. |
| 5 % Pd-C WET reduced Escat (STREM) | Feuchter und vorreduzierter Pd-Katalysator der Firma Strem mit der Markenbezeichnung Escat, der Aktivkohle als Trägermaterial enthält sowie einen Pd-Gehalt von 5 % und einen Wassergehalt von 50 % aufweist. |
| 10 % Pd-C WET reduced Escat (STREM) | Feuchter und vorreduzierter Pd-Katalysator der Firma Strem mit der Markenbezeichnung Escat, der Aktivkohle als Trägermaterial enthält sowie einen Pd-Gehalt von 10 % aufweist. |
| 5 % Pd-C WET reduced (AlfaAesar) | Feuchter und vorreduzierter Pd-Katalysator der Firma AlfaAesar mit einer BET-Oberfläche von 900 bis 1100 m²/g, der Aktivkohle als Trägermaterial enthält sowie einen Pd-Gehalt von 5 % und einen Wassergehalt von 50 % aufweist. |
| 5 % Pd on barium sulfate reduced (Aldrich) | Vorreduzierter Pd-Katalysator der Firma Aldrich, der Bariumsulfat als Trägermaterial enthält sowie einen Pd-Gehalt von 5% aufweist. |
| 5 % Pd on SiO₂ dry reduced Escat (STREM) | Vorreduzierter trockener Pd-Katalysator der Firma Strem mit der Markenbezeichnung Escat, der Siliziumoxid als Trägermaterial enthält sowie einen Pd-Gehalt von 5 % aufweist. |
| 3 % Pd SAP dry reduced (BASF-Italia) | Vorreduzierter trockener Pd-Katalysator der Firma BASF Italia, der ein Silizium/Aluminium-Mischoxid als Trägermaterial enthält sowie einen Pd-Gehalt von 3 % aufweist. |
| 5 % Pd on alumina reduced Escat (STREM) | Vorreduzierter Pd-Katalysator der Firma Strem mit der Markenbezeichnung Escat, der Aluminiumoxid als Trägermaterial enthält sowie einen Pd-Gehalt von 5 % aufweist. |
| 4 %Pd/4 %Au auf Aktivkohle | Vorreduzierter Pd-Katalysator (nicht kommerziell erhältliche Eigenproduktion der Firma BASF), der Aktivkohle als Trägermaterial enthält sowie einen Pd-Gehalt von 4 % und einen Au-Gehalt von 4 % aufweist. |

### I) Katalytische Isomerisierung:

Die einzelnen Versuche wurden in der Regel unter sauerstoffhaltiger Atmosphäre in einem druckstabilen Reaktor mit mechanischem Rührer durchgeführt. Zur Durchführung der katalytischen Isomerisierung im Labormaßstab wurde ein 350 ml Glasdruckgefäß (DN 50 für 8 bar), ausgestattet mit einem Strömungsbrecher, einer Heizvorrichtung (Huber Ministat 230-CC), einem Begasungsrührer (Pt 100, Sicherheitsventil 7 bar, Gasanschlüsse mit QC-Schnellkupplungen), einem Druckminderer für den Druckbereich 0-10 bar und gegebenenfalls einem Flowmeter am Gaseingang und einem Druckregler, d. h. Überströmer, am Gasausgang verwendet.

Falls nichts anderes angegeben ist, wurden für die nachfolgenden Versuche zur katalytischen Isomerisierung folgende Standardbedingungen verwendet:
- Rührgeschwindigkeit: 1000 U/min
- Austauschrate sauerstoffhaltiges Gas: 30 L/h
- Reaktionstemperatur: 80 °C
- Druck im Reaktor: 5-20 bar
- Eisatzstoffmenge: 100 g

Die Versuche zur katalytischen Isomerisierung wurden mit verschieden Pd-Katalysatoren durchgeführt. Die Resultate der Versuche sind in der Tabelle 1 zusammengefasst.

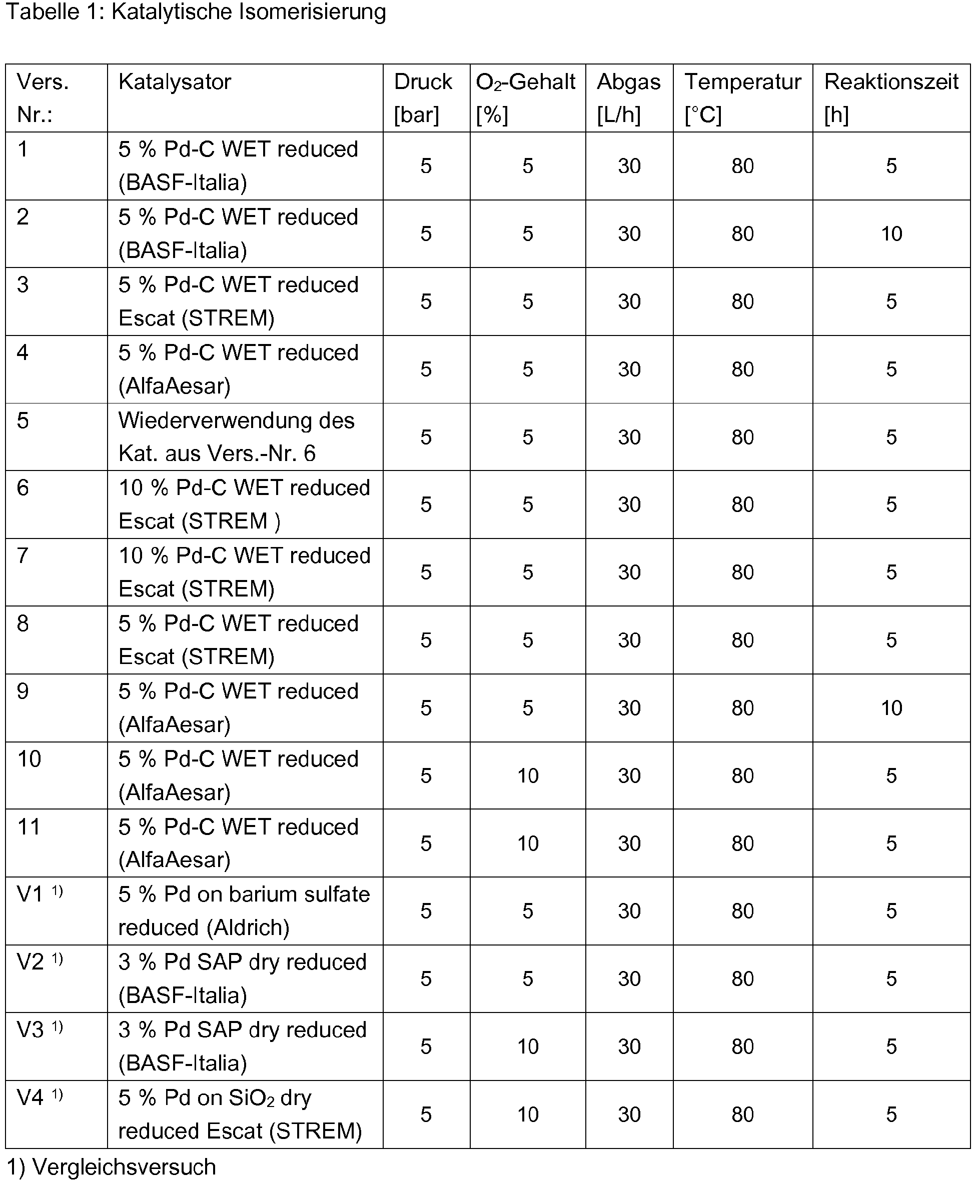

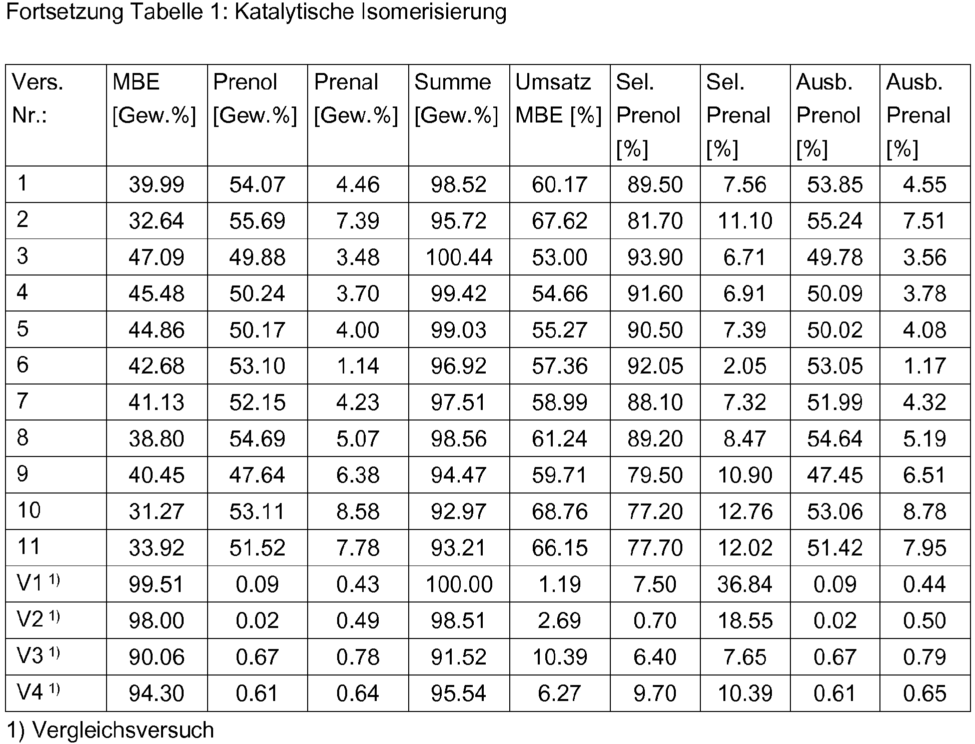

Wie aus der Tabelle 1 ersichtlich, zeigen Kohle-geträgerte Pd-Katalysatoren die gewünschte Aktivität. In der Regel werden Gesamtselektivitäten, d. h. die Selektivitäten von Prenol und Prenal zusammen, von über 90 % erreicht. Ein Recycling des Katalysators und anschließende Wiederverwendung konnte erfolgreich durchgeführt werden. Zudem konnte allgemein ein Rückgang der Bildung von Isoamylalkohol durch die verwendeten Reaktionsbedingungen festgestellt werden; die Werte lagen in einem Bereich von 0,5-1,0 FI.-%.

Tabelle 2 zeigt den zeitlichen Verlauf des Umsatzes an MBE sowie der Menge an gebildetem Prenol und Prenal einer repräsentativen katalytischen Isomerisierungsreaktion.

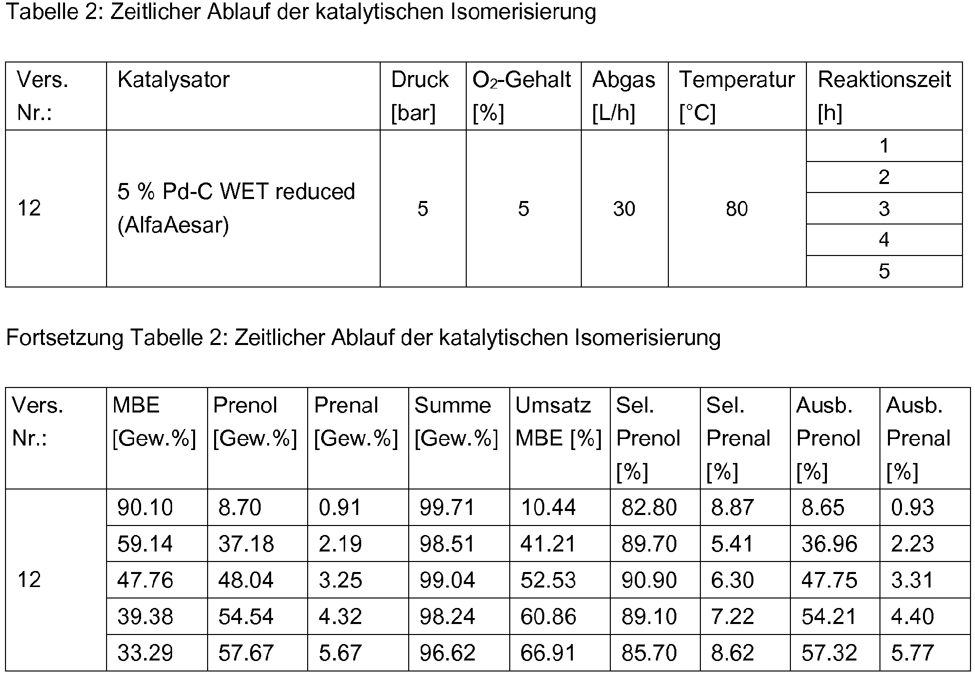

Wie der Tabelle 2 zu entnehmen ist, werden nach ca. 3 h Reaktionszeit die höchsten Werte für die Selektivität von Prenol erhalten bei einem MBE-Umsatz von über 50 %.

Die Austräge der Isomerisierung können ohne weitere Reinigung in der oxidativen Dehydrierung eingesetzt werden. Je nach Reaktionsverlauf und Reaktionsdauer ergeben sich dadurch Unterschiede in der Zusammensetzung der Reaktionseinsätze.

### II) Oxidative Dehydrierung von Austrägen aus der katalytischen Isomerisierung:

Als Einsatzstoff der nachfolgenden Versuche zur oxidativen Dehydrierung wurden Austräge aus der katalytischen Isomerisierung verwendet, die analog zu den unter I) aufgeführten Versuchen durchgeführt wurden. Dabei wurden die Isomerisierungsausträge nach Abtrennung des Katalysators direkt in die oxidative Dehydrierung eingesetzt, ohne eine Zwischenreinigung durchzuführen.

Die einzelnen Versuche zur oxidativen Dehydrierung wurden in der Regel unter sauerstoffhaltiger Atmosphäre in einem druckstabilen Reaktor mit mechanischem Rührer durchgeführt. Die Versuche wurden im Vergleich zur katalytischen Isomerisierung grundsätzlich bei einem höheren, bevorzugt bei einem 5 % höheren, O₂-Gehalt (10 Vol.-%) durchgeführt.

Falls nichts anderes angegeben ist, wurden für die nachfolgenden Versuche zur oxidativen Dehydrierung folgende Standardbedingungen verwendet:
- Rührgeschwindigkeit: 1000 U/min
- Austauschrate sauerstoffhaltiges Gas: 30 L/h
- Reaktionstemperatur: 80 °C
- Druck im Reaktor: 5-20 bar
- Eisatzstoffmenge: 100 g

Die Versuche zur oxidativen Dehydrierung der Isomerisierungsausträge von I) wurden mit den Katalysatoren 3 % Pd SAP dry reduced (BASF-Italia) und 4 %Pd/4 %Au auf Aktivkohle durchgeführt.

In den Tabellen 3 und 4 sind die Ergebnisse der Versuche zur oxidativen Dehydrierung unter direkter Verwendung von Austrägen aus katalytischen Isomerisierungen zusammengefasst. In den Tabellen 3 und 4 sind im oberen Teil der Zeile (t = 0) jeweils die Zusammensetzung der eingesetzten Isomerisierungsausträge aufgeführt, d. h. die für t = 0 angegebenen Werte entsprechen der Zusammensetzung des jeweils eingesetzten Isomerisierungsaustrags. Die unterschiedliche Zusammensetzung und Gesamtanteile an MBE, Prenol und Prenal der Isomerisierungsausträge ist auf unterschiedliche Reaktionsbedingungen, wie unterschiedliche Verweilzeiten, Durchführung eines Katalysatorrecyclings oder andere Variationen, bei der jeweils durchgeführte katalytischen Isomerisierung zurückzuführen.

**Tabelle 3: Oxidative Dehydrierung von Isomerisierungsausträgen. Katalysator: 3 % Pd SAP dry reduced (BASF-Italia); Zusatz: 0,2 % NaOH.**

| Vers. Nr.: | Einsatz: | Kat. Menge [g] | Druck [bar] | O₂-Gehalt [%] | Abgas [L/h] | Temperat ur [°C] | Reaktionszeit [h] |
|---|---|---|---|---|---|---|---|
| 13 | Austrag 1 | 1,25 (frisch) | 20 | 10 | 30 | 80 | 0 |
| | | | | | | | 2 |
| 14 | Austrag 2 | 1,25 (frisch) | 20 | 10 | 30 | 60 | 0 |
| | | | | | | | 5 |
| 15 | Austrag 3 | 1,25 (aus Vers.-Nr.15) | 20 | 10 | 30 | 60 | 0 |
| | | | | | | | 5 |
| 16 | Austrag 4 | 1,25 (aus Vers.-Nr.16) | 20 | 10 | 30 | 60 | 0 |
| | | | | | | | 2 |
| 17 | Austrag 5 | 1,25 (aus Vers.-Nr.16) | 20 | 10 | 30 | 70 | 0 |
| | | | | | | | 2 |
| 18 | Austrag 6 | 1,25 (aus Vers.-Nr.17) | 5 | 10 | 30 | 60 | 0 |
| | | | | | | | 5 |
| 19 | Austrag 7 | 1,25 (frisch) | 20 | 10 | 30 | 80 | 0 |
| | | | | | | | 2 |
| 20 | Austrag 8 | 1,25 (aus Vers.-Nr.20) | 20 | 10 | 30 | 80 | 0 |
| | | | | | | | 2 |

Fortsetzung Tabelle 3: Oxidative Dehydrierung von Isomerisierungsausträgen. Katalysator: 3 % Pd SAP dry reduced (BASF-Italia); Zusatz: 0,2 % NaOH.

| Vers. Nr.: | Prenol [Gew.%] | Prenal [Gew.%] | MBE [Gew.%] | Summe [Gew.%] | H₂O-Gehalt [%] | Umsatz MBE [%] | Sel. Prenol [%] | Sel. Prenal [%] | Sel. Gesamt [%] |
|---|---|---|---|---|---|---|---|---|---|
| 13 | 52.1 | 7 | 35.5 | 94.6 | n. b. | 67.3 | 80.5 | 10.2 | 90.7 |
| | 34.6 | 25.4 | 27.2 | 87.2 | 6.5 | 72.5 | 48.2 | 34.5 | 82.7 |
| 14 | 49.6 | 8.5 | 34.6 | 92.7 | n. b. | 65.4 | 75.8 | 12.7 | 89 |
| | 41.7 | 15.5 | 32.5 | 89.6 | 5.8 | 67.2 | 62.7 | 22.8 | 85 |
| 15 | 53.6 | 7.3 | 33.2 | 94.1 | n. b. | 66.8 | 80.2 | 10.7 | 90.9 |
| | 21.8 | 39.8 | 20.8 | 82.4 | n. b. | 78.5 | 28.9 | 51.5 | 80.3 |
| 16 | 52.7 | 7.4 | 38.8 | 98.9 | n. b. | 63.5 | 78 | 10.7 | 88.7 |
| | 35.7 | 23.4 | 27.9 | 86.9 | 7.7 | 73.1 | 47 | 30.1 | 77.1 |
| 17 | 54.1 | 7.6 | 33.8 | 95.5 | n. b. | 66.3 | 81.5 | 11.2 | 92.7 |
| | 33.8 | 25.9 | 24 | 83.7 | 9.1 | 75.3 | 46.2 | 34.6 | 80.8 |
| 18 | 42.9 | 50.1 | 5.1 | 98.1 | n. b. | 58.2 | 84 | 8.3 | 92 |
| | 35.7 | 23.3 | 29.4 | 88.3 | n. b. | 71.1 | 49.4 | 31.6 | 81 |
| 19 | 40.4 | 5.4 | 52.3 | 98.1 | n. b. | 47.8 | 84.7 | 11 | 95.7 |
| | 32.5 | 11.2 | 45.9 | 89.6 | 2.3 | 54.2 | 62.6 | 21.1 | 83.7 |
| 20 | 46 | 6.6 | 40.8 | 93.4 | n. b. | 59.3 | 77.4 | 10.8 | 88.2 |
| | 28.9 | 26.2 | 28.84 | 83.9 | 7.7 | 71.3 | 42.9 | 38 | 80.9 |

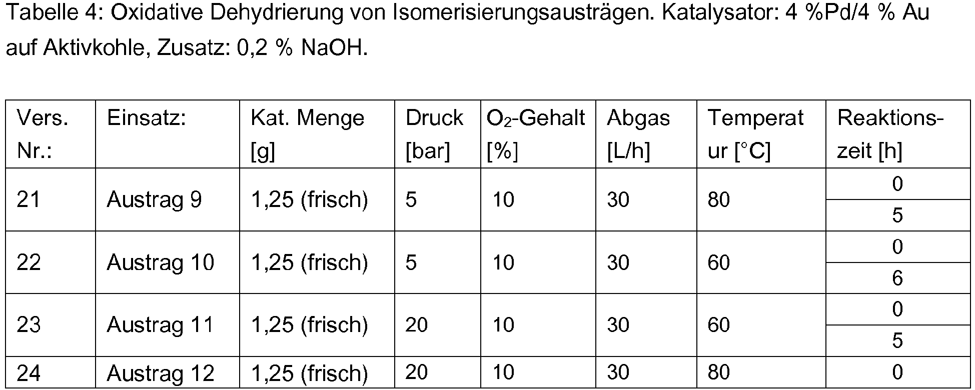

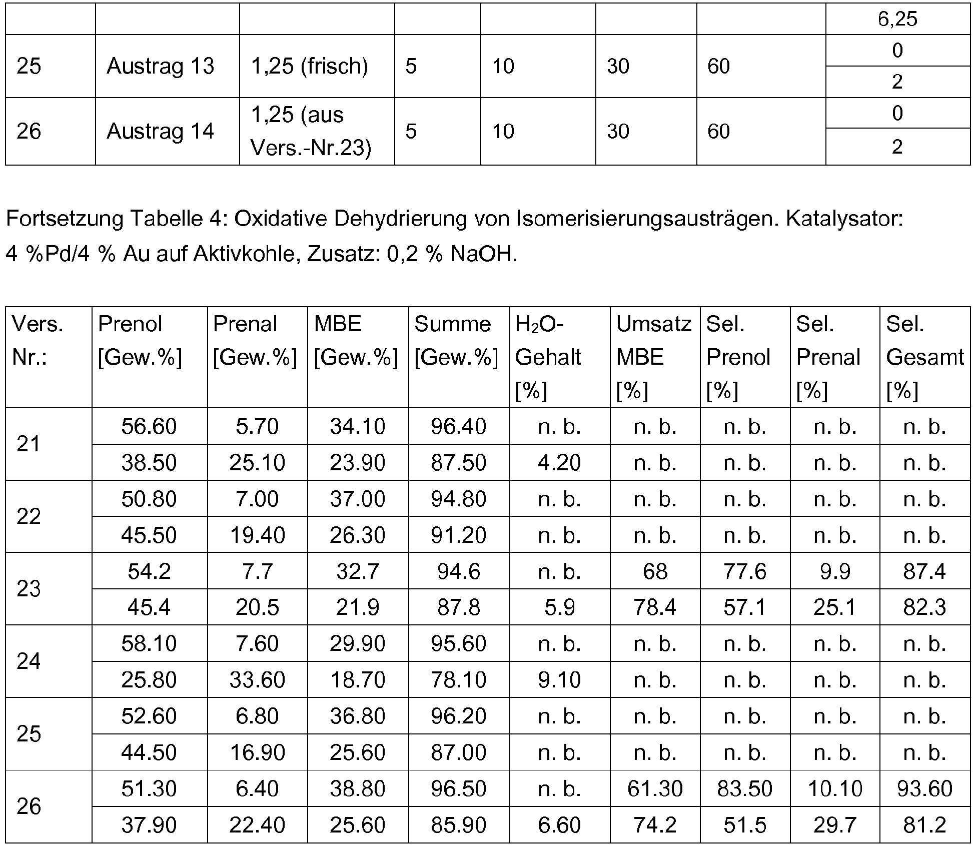

Der direkte Einsatz der Austräge aus der katalytischen Isomerisierung in die oxidative Dehydrierung ist möglich, da bei der katalytischen Isomerisierung offensichtlich kein Katalysatorgift gebildet wird. Recycling-Versuche (bis zu 4 x) waren sowohl für den verwendeten 3 % Pd SAP-Katalysator als auch für den 4 % Pd/4 % Au-Katalysator erfolgreich. Es kann hierbei sogar eine Zunahme der Aktivität beobachtet werden. Die Anhebung von Temperatur, Druck und Verweilzeit führt im Allgemeinen zu einer Umsatzsteigerung, jedoch ist aufgrund von Zersetzungs- und Folgereaktionen, beispielsweise einer Überoxidation, mit Selektivitätseinbußen zu rechnen.

### III) Oxidative Dehydrierung einer Mischung aus Prenol und MBE und von reinem Prenol:

In der Tabelle 6 sind die Ergebnisse aus Versuchen zur oxidativen Dehydrierung einer spezifischen Mischung aus Prenol und MBE und als Vergleichsversuch von reinem Prenol. Die in der Tabelle 5 unter t = 0 aufgeführten Werte entsprechen der Menge des jeweils verwendeten Einsatzstoffes oder der Zusammensetzung der jeweils verwendeten Einsatzstoffe.

3 % Pd SAP dry reduced (BASF-Italia) kam als Katalysator zum Einsatz. Alle Versuche wurden mit 30 L/h Abgas und 0,2 % NaOH gefahren. Ansonsten wurden die unter II) angegebenen Standardbedingungen verwendet. Die angegebene Gesamtselektivität entspricht der Summe aus den Selektivitäten von Prenol und Prenal.

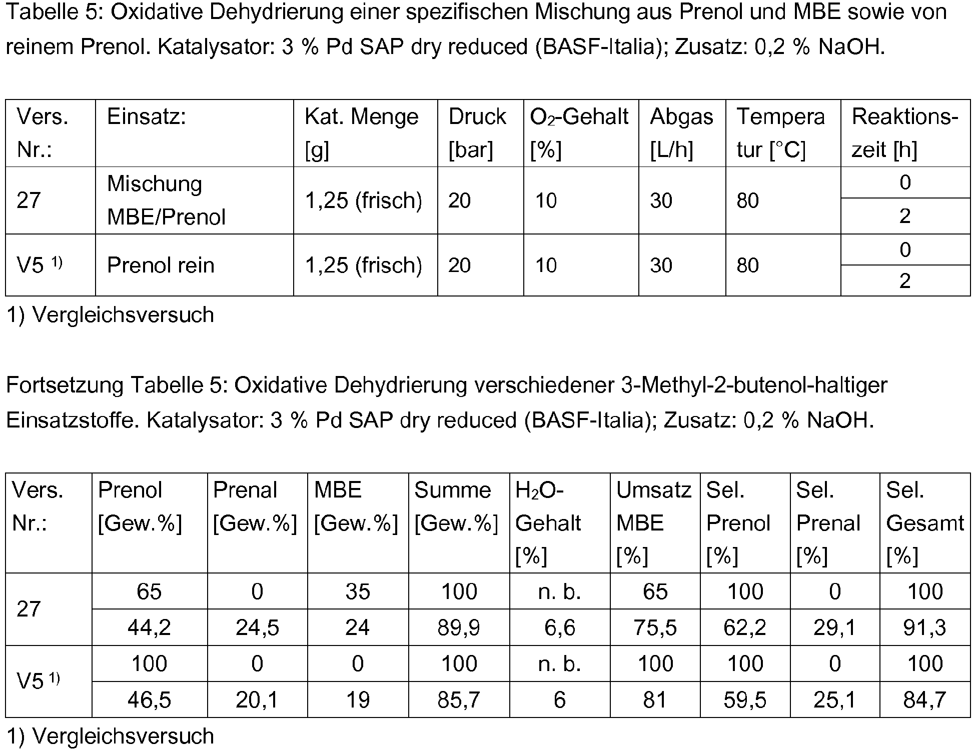

Die Versuche zeigen, dass mit Mischungen von MBE/Prenol (rein) hohe Gesamtselektivitäten (91 %) in der oxidativen Dehydrierung erreicht werden können. Die Verwendung von reinem Prenol hingegen führte lediglich zu einer Gesamtselektivität von ca. 85 %. Dies deckt sich mit den Erfahrungen aus Blindversuchen, die zeigten, dass sich Prenol in Anwesenheit von Sauerstoff sehr schnell zersetzen kann. Der Einsatz des Originalaustrags aus den katalytischen Isomerisierungen liefert vergleichbare Ergebnisse wie eine synthetische MBE/Prenol-Mischung. Der Einsatz einer MBE/Prenol-Mischung erweist sich somit als vorteilhaft.

## Patentansprüche

1. Verfahren zur Herstellung einer Zusammensetzung, die 3-Methyl-2-butenol und 3-Methyl-2-butenal enthält, bei dem man
i) 3-Methyl-3-butenol einer katalytischen Isomerisierung an einem Kohlenstoff-geträgerten Pd-Katalysator in Gegenwart eines Gasgemisches enthaltend 1 bis 15 Vol.-% Sauerstoff unterzieht, unter Erhalt eines ersten Produktgemisches, das wenigstens 40 Gew.-% 3-Methyl-2-butenol, wenigstens 5 Gew.-% 3-Methyl-3-butenol und 0 bis 15 Gew.-% 3-Methyl-2-butenal, bezogen auf das Gesamtgewicht des ersten Produktgemisches, enthält,
ii) das in Schritt i) erhaltene erste Produktgemisch einer oxidativen Dehydrierung an einem Pd-Katalysator, der SiO₂ und/oder Al₂O₃ als Trägermaterial enthält, oder an einem Kohlenstoff-geträgerten Pd/Au-Katalysator in Gegenwart eines Gasgemisches enthaltend 5 bis 25 Vol.-% Sauerstoff unterzieht, unter Erhalt eines gegenüber dem ersten Produktgemisch an 3-Methyl-2-butenal angereicherten und 3-Methyl-2-butenol abgereicherten zweiten Produktgemisches, wobei das molare Verhältnis von 3-Methyl-2-butenol zu 3-Methyl-2-butenal in dem zweiten Produktgemisch im Bereich von 75 : 25 bis 35 : 65 liegt,
wobei die oxidative Dehydrierung in Schritt ii) zusätzlich in Gegenwart einer Base durchgeführt wird.

2. Verfahren nach Anspruch 1, wobei als Katalysator in Schritt ii) ein Pd-Katalysator, der SiO₂ und/oder Al₂O₃ als Trägermaterial enthält, eingesetzt wird.

3. Verfahren nach einem der vorangehenden Ansprüche 1 oder 2, wobei das in Schritt i) eingesetzte Gasgemisch 3 bis 10 Vol.-% Sauerstoff enthält.

4. Verfahren nacheinem der vorangehenden Ansprüche, wobei das in Schritt ii) eingesetzte Gasgemisch 8 bis 15 Vol.-% Sauerstoff enthält.

5. Verfahren nach einem der vorangehenden Ansprüche, wobei der Sauerstoffgehalt des in Schritt i) eingesetzten Gasgemisches 2 bis 10 Vol.-% unter dem Sauerstoffgehalt des in Schritt ii) eingesetzten Gasgemisches liegt.

6. Verfahren nach einem der vorangehenden Ansprüche, wobei das in Schritt i) erhaltene erste Produktgemisch aus
i.1 40 bis 80 Gew.-% 3-Methyl-2-butenol,
i.2 0 bis 15 Gew.-% 3-Methyl-2-butenal,
i.3 5 bis 59 Gew.-% 3-Methyl-3-butenol und
i.4 0 bis 10 Gew.-% von i.1, i.2 und i.3 verschiedenen Verbindungen besteht.

7. Verfahren nach einem der vorangehenden Ansprüche, wobei das in Schritt i) erhaltene erste Produktgemisch aus
i.1 45 bis 70 Gew.-% 3-Methyl-2-butenol,
i.2 5 bis 10 Gew.-% 3-Methyl-2-butenal,
i.3 20 bis 50 Gew.-% 3-Methyl-3-butenol und
i.4 0 bis 5 Gew.-% von i.1, i.2 und i.3 verschiedenen Verbindungen besteht.

8. Verfahren nach einem der vorangehenden Ansprüche, wobei der Gesamtanteil an 3-Methyl-2-butenol, 3-Methyl-2-butenal und 3-Methyl-3-butenol in dem in Schritt i) erhaltene ersten Produktgemisch wenigstens 80 Gew.-%, bezogen auf das Gesamtgewicht des ersten Produktgemisches, beträgt.

9. Verfahren nach einem der vorangehenden Ansprüche, wobei Schritt i) ohne Zuführung eines externen Lösungsmittels durchgeführt wird.

10. Verfahren nach einem der vorangehenden Ansprüche, wobei man die katalytische Isomerisierung von 3-Methyl-3-butenol in Gegenwart von Sauerstoff bis zu einem Umsatz von 30 bis 80 %, bezogen auf das eingesetzte 3-Methyl-3-butenol, führt.

11. Verfahren nach einem der vorangehenden Ansprüche, wobei das Verfahren eines oder mehrere der folgenden Merkmale a) bis c) aufweist:
a) der Austrag der katalytischen Isomerisierung wird, gegebenenfalls nach Abtrennung des Katalysators, ohne weitere Aufreinigung in die oxidative Dehydrierung des Schrittes ii) eingesetzt,
b) der Anteil der Base in der Reaktionsmischung beträgt 0,01 bis 2 Gew.-%, bezogen auf das Gesamtgewicht des in Schritt ii) eingesetzten ersten Produktgemisches,
c) Schritt ii) wird ohne Zuführung eines externen Lösungsmittels durchgeführt.

12. Verfahren nach einem der vorangehenden Ansprüche, wobei in den Schritten i) und ii) ein an Sauerstoff angereichertes Gasgemisch den jeweiligen Reaktionszonen kontinuierlich zugeführt und ein an Sauerstoff abgereichertes Gasgemisch kontinuierlich abgeführt wird, wobei die Zuführung des an Sauerstoff angereicherten Gasgemisches unterhalb der Flüssigkeitsoberfläche der jeweiligen Reaktionsgemische erfolgt.

13. Verfahren nach einem der vorangehenden Ansprüche, wobei das Verfahren kontinuierlich durchgeführt wird.

14. Verfahren nach einem der vorangehenden Ansprüche, wobei man das in Schritt ii) erhaltene zweite Produktgemisch einer destillativen Auftrennung in eine an 3-Methyl-2-butenol und 3-Methyl-2-butenal angereicherte Produktfraktion und eine an 3-Methyl-3-butenol angereicherte Eduktfraktion unterzieht.

15. Verfahren nach Anspruch 14, wobei die an 3-Methyl-3-butenol angereicherte Eduktfraktion, gegebenenfalls unter Zusatz von frischem 3-Methyl-3-butenol, in Schritt i) eingesetzt wird.

## Claims

1. A process for preparing a composition comprising 3-methyl-2-butenol and 3-methyl-2-butenal, in which
i) 3-methyl-3-butenol is subjected to a catalytic isomerization over a carbon-supported Pd catalyst in the presence of a gas mixture comprising 1% to 15% by volume of oxygen to obtain a first product mixture comprising at least 40% by weight of 3-methyl-2-butenol, at least 5% by weight of 3-methyl-3-butenol and 0% to 15% by weight of 3-methyl-2-butenal, based on the total weight of the first product mixture,
ii) the first product mixture obtained in step i) is subjected to an oxidative dehydrogenation over a Pd catalyst comprising SiO₂ and/or Al₂O₃ as support material, or over a carbon-supported Pd/Au catalyst in the presence of a gas mixture comprising 5% to 25% by volume of oxygen, to obtain a second product mixture which is enriched in 3-methyl-2-butenal and depleted in 3-methyl-2-butenol compared to the first product mixture, where the molar ratio of 3-methyl-2-butenol to 3-methyl-2-butenal in the second product mixture is in the range from 75:25 to 35:65, wherein the oxidative dehydrogenation in step ii) is additionally conducted in the presence of a base.

2. The process according to claim 1, wherein the catalyst used in step ii) is a Pd catalyst comprising SiO₂ and/or Al₂O₃ as support material.

3. The process according to either of the preceding claims 1 and 2, wherein the gas mixture used in step i) comprises 3% to 10% by volume of oxygen.

4. The process according to any of the preceding claims, wherein the gas mixture used in step ii) comprises 8% to 15% by volume of oxygen.

5. The process according to any of the preceding claims, wherein the oxygen content of the gas mixture used in step i) is 2% to 10% by volume below the oxygen content of the gas mixture used in step ii).

6. The process according to any of the preceding claims, wherein the first product mixture obtained in step i) consists of
i.1 40% to 80% by weight of 3-methyl-2-butenol,
i.2 0% to 15% by weight of 3-methyl-2-butenal,
i.3 5% to 59% by weight of 3-methyl-3-butenol and
i.4 0% to 10% by weight of compounds other than i.1, i.2 and i.3.

7. The process according to any of the preceding claims, wherein the first product mixture obtained in step i) consists of
i.1 45% to 70% by weight of 3-methyl-2-butenol,
i.2 5% to 10% by weight of 3-methyl-2-butenal,
i.3 20% to 50% by weight of 3-methyl-3-butenol and
i.4 0% to 5% by weight of compounds other than i.1, i.2 and i.3.

8. The process according to any of the preceding claims, wherein the total proportion of 3-methyl-2-butenol, 3-methyl-2-butenal and 3-methyl-3-butenol in the first product mixture obtained in step i) is at least 80% by weight, based on the total weight of the first product mixture.

9. The process according to any of the preceding claims, wherein step i) is conducted without supply of an external solvent.

10. The process according to any of the preceding claims, wherein the catalytic isomerization of 3-methyl-3-butenol in the presence of oxygen is run up to a conversion of 30% to 80%, based on the 3-methyl-3-butenol used.

11. The process according to any of the preceding claims, wherein the process has one or more of the following features a) to c):
a) the output from the catalytic isomerization, optionally after removal of the catalyst, is used without further purification in the oxidative dehydrogenation in step ii),
b) the proportion of the base in the reaction mixture is 0.01% to 2% by weight, based on the total weight of the first product mixture used in step ii),
c) step ii) is conducted without supply of an external solvent.

12. The process according to any of the preceding claims, wherein, in steps i) and ii), an oxygen-enriched gas mixture is supplied continuously to the respective reaction zones and an oxygen-depleted gas mixture is removed continuously, wherein the supply of the oxygen-enriched gas mixture is below the liquid surface of the respective reaction mixtures.

13. The process according to any of the preceding claims, which is performed continuously.

14. The process according to any of the preceding claims, wherein the second product mixture obtained in step ii) is subjected to a distillative separation into a product fraction enriched in 3-methyl-2-butenol and 3-methyl-2-butenal and a reactant fraction enriched in 3-methyl-3-butenol.

15. The process according to claim 14, wherein the reactant fraction enriched in 3-methyl-3-butenol, optionally with addition of fresh 3-methyl-3-butenol, is used in step i).

## Revendications

1. Procédé pour la préparation d'une composition, qui contient du 3-méthyl-2-buténol et du 3-méthyl-2-buténal, dans lequel on
i) soumet du 3-méthyl-3-buténol à une isomérisation catalytique sur un catalyseur de Pd supporté sur du carbone en présence d'un mélange de gaz contenant 1 à 15 % en volume d'oxygène, avec obtention d'un premier mélange de produits, qui contient au moins 40 % en poids de 3-méthyl-2-buténol, au moins 5 % en poids de 3-méthyl-3-buténol et zéro à 15 % en poids de 3-méthyl-2-buténal par rapport au poids total du premier mélange de produits,
ii) on soumet le premier mélange de produit obtenu dans l'étape i) à une déshydratation oxydante sur un catalyseur de Pd, qui contient SiO₂ et/ou Al₂O₃ en tant que matériau de support, ou sur un catalyseur de Pd/Au supporté sur du carbone en présence d'un mélange de gaz contenant 5 à 25 % en volume d'oxygène, avec obtention d'un deuxième mélange de produits enrichi en 3-méthyl-2-buténal et appauvri en 3-méthyl-2-buténol par rapport au premier mélange de produits, le rapport molaire de 3-méthyl-2-buténol à 3-méthyl-2-buténal dans le deuxième mélange de produits se situant dans la plage de 75:25 à 35:65,
la déshydratation oxydante dans l'étape ii) étant de plus mise en œuvre en présence d'une base.

2. Procédé selon la revendication 1, un catalyseur de Pd, qui contient SiO₂ et/ou Al₂O₃ en tant que matériau de support étant utilisé en tant que catalyseur dans l'étape ii).

3. Procédé selon l'une quelconque des revendications précédentes 1 et 2, le mélange de gaz utilisé dans l'étape i) contenant 3 à 10 % en volume d'oxygène.

4. Procédé selon l'une quelconque des revendications précédentes, le mélange de gaz utilisé dans l'étape ii) contenant 8 à 15 % en volume d'oxygène.

5. Procédé selon l'une quelconque des revendications précédentes, la teneur en oxygène du mélange de gaz utilisé dans l'étape i) se situant de 2 à 10 % en volume en dessous de la teneur en oxygène du mélange de gaz utilisé dans l'étape ii).

6. Procédé selon l'une quelconque des revendications précédentes, le premier mélange de produits obtenu dans l'étape i) étant constitué de
i.1 40 à 80 % en poids de 3-méthyl-2-buténol,
i.2 0 à 15 % en poids de 3-méthyl-2-buténal,
i.3 5 à 59 % en poids de 3-méthyl-3-buténol et
i.4 0 à 10 % en poids de composés différents de i.1, i.2 et i.3.

7. Procédé selon l'une quelconque des revendications précédentes, le premier mélange de produits obtenu dans l'étape i) étant constitué de
i.1 45 à 70 % en poids de 3-méthyl-2-buténol,
i.2 5 à 10 % en poids de 3-méthyl-2-buténal,
i.3 20 à 50 % en poids de 3-méthyl-3-buténol et
i.4 0 à 5 % en poids de composés différents de i.1,
i.2 et i.3.

8. Procédé selon l'une quelconque des revendications précédentes, la proportion totale en 3-méthyl-2-buténol, 3-méthyl-2-buténal et 3-méthyl-3-buténol dans le premier mélange de produits obtenu dans l'étape i) étant d'au moins 80 % en poids, par rapport au poids total du premier mélange de produits.

9. Procédé selon l'une quelconque des revendications précédentes, l'étape i) étant mise en œuvre sans introduction d'un solvant externe.

10. Procédé selon l'une quelconque des revendications précédentes, l'isomérisation catalytique de 3-méthyl-3-buténol étant conduite en présence d'oxygène jusqu'à une conversion de 30 à 80 %, par rapport au 3-méthyl-3-buténol utilisé.

11. Procédé selon l'une quelconque des revendications précédentes, le procédé présentant une ou plusieurs des caractéristiques suivantes a) à c) :
a) le produit de l'isomérisation catalytique, éventuellement après la séparation du catalyseur, est utilisé sans autre purification dans la déshydratation oxydante de l'étape ii),
b) la proportion de base dans le mélange réactionnel est de 0,01 à 2 % en poids, par rapport au poids total du premier mélange de produits utilisé dans l'étape ii),
c) l'étape ii) est mise en œuvre sans introduction d'un solvant externe.

12. Procédé selon l'une quelconque des revendications précédentes, où dans les étapes i) et ii) un mélange de gaz enrichi en oxygène est introduit en continu dans les zones de réaction respectives et un mélange de gaz appauvri en oxygène est évacué en continu, l'introduction du mélange de gaz enrichi en oxygène étant réalisée au-dessous de la surface liquide des mélanges réactionnels respectifs.

13. Procédé selon l'une quelconque des revendications précédentes, le procédé étant mis en œuvre en continu.

14. Procédé selon l'une quelconque des revendications précédentes, où on soumet le deuxième mélange de produits obtenu dans l'étape ii) à une séparation par distillation en une fraction de produit enrichie en 3-méthyl-2-buténol et 3-méthyl-2-buténal et en une fraction d'éduit enrichie en 3-méthyl-3-buténol.

15. Procédé selon la revendication 14, la fraction d'éduit enrichie en 3-méthyl-3-buténol étant utilisée dans l'étape i), éventuellement avec ajout de 3-méthyl-3-buténol frais.
